# EUROPEAN PATENT SPECIFICATION

(11) **EP 3 206 563 B1**
(45) Date of publication and mention of the grant of the patent: **09.05.2018**
(21) Application number: 15771902.2
(22) Date of filing: 23.09.2015
(51) Int. Cl.: A61B 5/00, A61B 5/113, A61B 5/08, G06K 9/00

(54) **DEVICE AND METHOD FOR DETECTING VITAL SIGN INFORMATION OF A SUBJECT**
SYSTEM UND VERFAHREN ZUR ERKENNUNG DER LEBENSZEICHENINFORMATIONEN EINES PATIENTEN
SYSTÈME ET PROCÉDÉ PERMETTANT DE DÉTERMINER LES INFORMATIONS DE SIGNES VITAUX D'UNE PERSONNE

(30) Priority: 13.10.2014 EP 14188574
(43) Date of publication of application: 23.08.2017
(73) Proprietor: Koninklijke Philips N.V., 5656 AE Eindhoven (NL)
(72) Inventor: KIRENKO, Ihor Olehovych, 5656 AE Eindhoven (NL)
(74) Representative: de Haan, Poul Erik
(86) International application number: PCT/EP2015/071820
(87) International publication number: WO 2016/058796

(56) References cited:
- WO-A1-2013/164724
- WO-A1-2014/020509
- JP-A- 2006 187 510
- US-A1- 2009 141 124

## Description

### FIELD OF THE INVENTION

The present invention relates to a device and a corresponding method for detecting vital sign information of a subject. The present invention is in particular directed to a remote measurement of a respiration rate or a pulse rate of a subject.

### BACKGROUND OF THE INVENTION

Vital signs of a person, for example the respiration rate or the pulse rate (sometimes also called heart rate), serve as indicators for the current state of the person and as predictions of serious medical events. For this reason, vital signs are extensively monitored in inpatient and outpatient care settings, at home or in further health, leisure and fitness settings.

Camera-based monitoring of the vital signs for example the respiration rate or the pulse rate is a known technique for fully contactless measuring the vital signs of a person. Since the subject of interest, i.e. the person to be measured can be located freely in the field of view of the camera, the relevant area from which the respective vital sign information should be acquired has to be defined as the input for the expectation of the respective signals. Further, cameras provide 2D information, which allows for a multi-spot and large area measurement, and often contains additional context information. Unlike with contact sensors which rely on the correct placement on the relevant measurement point/area, the region of interest (ROI) used to measure the vital sign, e.g. a respiratory signal or a pulse rate signal, has to be determined from the actual image.

In most applications for contactless vital sign measurements, the region of interest is selected manually or the used camera is directed to the region of interest in advance, however, a movement of the subject leads to incorrect measurements and an impractical use of the system. Therefore, an automatic detection of the region of interest is desired to improve the camera-based monitoring of the vital sign information.

Conventional methods to determine the region of interest for respiration rate or heart rate detection on the basis of contour detection such as face detection are e.g. disclosed in US 2009/0141124 A1. The disadvantage of this method is that the region of interest cannot be detected reliable, if the contour, i.e. the face is partially or fully occluded or hidden when the respective portion of the subject to be measured is covered by a blanket, which is a typical case in hospitals where a monitoring of the respiration rate and pulse rate is critical.

Other methods which are based on a shape analysis, such as chest/thorax detection, for the detection of the region of interest are limited by the position of the subject within the field of view or by the worn clothing so that those detection methods are less reliable.

A method for identifying a region of interest for respiration monitoring is for example known from EP 0919184 A1, whereas the region of interest is determined on the basis of changed portions between successive images captured from the field of view, wherein the changes between successive images can be based on disturbance signals, which do not refer to vital signs. Hence, the method known from this document is less reliable.

A further method for monitoring the respiration of a subject is known from US 7431700 B2, wherein a time based change in the image data is analyzed and a periodic appearance is detected as respiration, however, since all time based changes in the whole field of view are considered and no region of interest is detected, the presence of disturbing signals can lead to incorrect measurements of the respiration rate. Hence, the method disclosed in this document is less reliable and has an increased technical effort.

Still further, WO 2014/131850 A1 discloses an apparatus for determining vital sign information from a subject, comprising a detection unit for detecting radiation from a field of view and for determining characteristic parameter including vital sign information of the subject from different areas of the field of view, a frequency analysis unit for determining a spectral parameter of the characteristic parameter derived from the different areas, a selection unit for selecting at least one of the areas of the field of view on the basis of the spectral parameter, and a calculation unit for calculating the vital sign information on the basis of the characteristic parameter from the at least one selected area.

The disadvantage of some of the known methods to detect a region of interest for remotely detection vital sign information from a subject is that the whole image detected from the field of view is used to detect the vital sign information so that these methods are susceptible to disturbance signals in the field of view and to movements of the subject within the field of view so that the known methods for determining vital signs from the subject are less reliable. Further, in case of measurement of respiratory information, in the absence of a respiratory signal (e.g. due to apnea moments) the ROI would not be detectable, or would be lost. Thus, reliable monitoring of vital signs of a patient with apnea is not possible. Still further, after motion of a body, the previously selected ROI is not optimal or valid. Therefore, a new ROI should be re-initialized, which might require a significant amount of time due to the analysis of a temporal vital signal.

### SUMMARY OF THE INVENTION

It is an object of the present invention to provide an improved apparatus and a corresponding method for detecting vital sign information of a subject, where these are less susceptible to disturbing signals, movements of the subject to be measured or provide a higher responsiveness, accuracy or reliability.

According to one aspect of the present invention, a device for determining vital sign information of a subject is provided, comprising:
- an input unit for receiving image data of the subject, said image data including a sequence of images over time,
- an ROI selection unit for selecting an initial region of interest, ROI, within an image of the sequence of images, said initial ROI comprising a body part of the subject,
- a feature selection unit for selecting one or more spatial features within the initial ROI,
- a motion signal extraction unit for extracting from said image data the direction and/or amplitude of motion of the body part related to a desired vital sign of the subject,
- a detection unit for detecting one or more spatial features of said selected spatial features, whose motion is not related to the desired vital sign of the subject, based on the extracted direction and/or amplitude of motion of the body part,
- a tracking unit for tracking the initial ROI by detecting changes of the position of said one or more detected spatial features and by shifting the position of the initial ROI based on the detected changes to obtain a final ROI, and
- a vital signs extraction unit for extracting the desired vital sign from the final ROI within images of said sequence of images.

According to another aspect of the present invention, a corresponding method for determining vital sign information of a subject is provided.

In yet further aspects of the present invention, there are provided a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed methods, processor, computer program and medium have similar and/or identical preferred embodiments as the claimed device and as defined in the dependent claims.

The present invention is based on the idea to improve the device and method described in the above mentioned WO 2014/131850 A1 by using the strengths of this device and method by additionally selecting and tracking of spatial features of a body part of the subject with an ROI selected beforehand. This provides for an improvement of the accuracy and SNR of the extracted vital signs and for a fast adaptation and tracking of the ROI.

The known device and method for automatic detection of ROI for measurement of vital sign information, in particular respiratory signals, is based on a temporal analysis of spatial blocks and selection of spatial areas, which produce temporal signal with characteristics (amplitude, periodicity), which satisfy the predefined description of an expected vital sign signal, such as a respiratory signal or pulse-induced motion. Such approach is very efficient for detection of only those areas, which produce strong vital sign signal. For instance, detection of the ROI based on selection of spatial blocks with the strongest respiratory motion significantly improves the quality of the extract signal, compared to measurement of respiratory signal from a ROI selected based on other criteria.

This approach is improved by tracking the ROI for extraction of a vital sign by selecting spatial features (also called feature points) within a pre-selected spatial area, i.e. within an initial ROI, and applying motion detection for detecting motion of one or more of said spatial features which is not influenced by vital signs unrelated motion of the body part, i.e. where the motion of the spatial features is not caused by vital signs related motion, such as motion of the chest caused by respiration or motion of a head caused by heartbeat. Motion estimation or pattern matching techniques may then be used to adapt the position of the (initial) ROI based on the motion of the detected spatial features, which is not caused by the motion of the body part related to the vital signs. Thus, a new ROI (called final ROI) is obtained through this tracking, which tracking of the ROI can be very fast and accurate. Further, this allows detection of the absence of a vital signs signal (e.g. an apnea moment) from a stable ROI.

In other words, according to the present invention detection and tracking of the ROI are based on a combined analysis of the measured vital sign and on spatial features invariant to the measured vital sign. Spatial features inside the initial ROI, which are not affected by the vital sign, are used for this purpose. For instance, if there is a lateral motion of a head due to heartbeat, the distances between eyes or other face features may be used as that spatial feature in one exemplary embodiment.

In a preferred embodiment the device further comprises an ROI re-initialization unit for re-initializing the final ROI as new initial ROI. Hence, the proposed method for ROI detection and tracking can be continuously and iteratively performed in order to continuously (or regularly) monitor and track movements of the ROI.

In another embodiment said tracking unit is configured to use as final ROI the ROI obtained from tracking of the initial ROI after the position of the one or more spatial feature has become stable over several image frames. In this way the vital signs extracted from the final ROI will be more accurate and reliable.

In another embodiment said feature selection unit is configured to select one or more spatial features within the initial ROI, which are invariant, preferably most invariant, to motion of a subject's body part related to a desired vital sign of the subject. This will make it more easy and precise to distinguish movements reflecting the desired vital sign from movements which have nothing to do with the vital sign and are e.g. caused by motion of the patient.

In a preferred implementation the motion of a subject's body part related to a desired vital sign of the subject is breathing motion and the vital sign information is respiratory information. In another preferred implementation the motion of a subject's body part related to a desired vital sign of the subject is heartbeat-induced motion and the vital sign information is pulse rate information. Breathing motion can e.g. be detected from chest or belly movements, and heartbeat motion can e.g. be detected from movements of left chest or even of the head (as currently e.g. is described at http://newsoffice.mit.edu/2013/seeing-the-human-pulse-0620).

Preferably, in such implementations said feature selection unit is configured to select one or more spatial features within the initial ROI, which are invariant, in particular most invariant, to breathing motion or heartbeat motion, in particular spatial features showing edges or lines in an image within the initial ROI arranged along the main direction of said breathing motion or heartbeat motion within the initial ROI. The main direction of said breathing motion or heartbeat motion can e.g. be determined by the motion signal extraction unit. For instance, after applying a motion estimation algorithm to pixels within an initial ROI, optical flow or/and motion vectors are analyzed. Based on the analysis of the obtained motion vectors, the main direction, which correlated the most with the vital sign signal, is defined. For instance, in case of respiratory signal extraction from a person sitting in front of a camera, respiratory motion will be correlated the most with vertical motion vectors, while horizontal vectors, will not correspond to respiratory motion.

Advantageously, said detection unit is configured to detect one or more spatial features of said selected spatial features, whose motion is not related to the desired vital sign of the subject, by detecting changes of the position of said selected spatial features within the initial ROI and the extracted direction and/or amplitude of motion of a subject's body part related to a desired vital sign of the subject. Preferably, changes of distances between selected spatial features within the initial ROI, the direction of changes of the position of selected spatial features within the initial ROI and/or the deviation of changes of the position of selected spatial features within the initial ROI from the motion of a subject's body part related to a desired vital sign of the subject are analyzed. This provides a simple but effective way for detecting such spatial features.

In an embodiment said ROI selection unit is configured to select the initial ROI within an image by dividing the image into spatial blocks, extracting a vital sign from a plurality of said spatial blocks, clustering of two or more adjacent spatial blocks, from which the strongest vital signs are extracted, and selecting said clustered spatial blocks as initial ROI. This provides an optimal initial ROI.

In a further embodiment said ROI selection unit is configured to select the initial ROI from a previously obtained final ROI by extracting a vital sign from a plurality of spatial blocks adjacent to the obtained final ROI, clustering of two or more adjacent spatial blocks, from which the strongest vital signs are extracted, and selecting said clustered spatial blocks as new initial ROI. This improves the quality of the initial ROI.

Preferably, said ROI selection unit is configured to select the initial ROI from a previously obtained final ROI if the reliability or quality of the vital sign from the final ROI drops below a predetermined threshold or by predetermined amount. Thus, another iteration of the proposed method for obtaining a new final ROI is performed to improve the reliability or quality of the vital sign derived from the previous final ROI.

In another embodiment said motion detection unit is configured to detect if the initial ROI is at least partly occluded and said vital signs extraction unit is configured to extract the desired vital sign from non-occluded parts of the initial ROI. This also improves the accuracy of the extracted vital sign.

### BRIEF DESCRIPTION OF THE DRAWINGS

These and other aspects of the invention will be apparent from and elucidated with reference to the embodiments described hereinafter. In the following drawings
Fig. 1 shows a schematic illustration of a general layout of a system and device for determining vital sign information of a subject according to the present invention,
Fig. 2 shows a schematic illustration of a subject's motion indicative of exemplary vital sign information,
Fig. 3 shows a timing diagram of an alternating signal derived from the subject corresponding to the vital sign information,
Fig. 4 shows a flow chart of an embodiment of the method according to the present invention, and
Figs. 5a, 5b, and 5c show a schematic image sequence for illustrating the steps of the method according to the present invention.

### DETAILED DESCRIPTION OF THE INVENTION

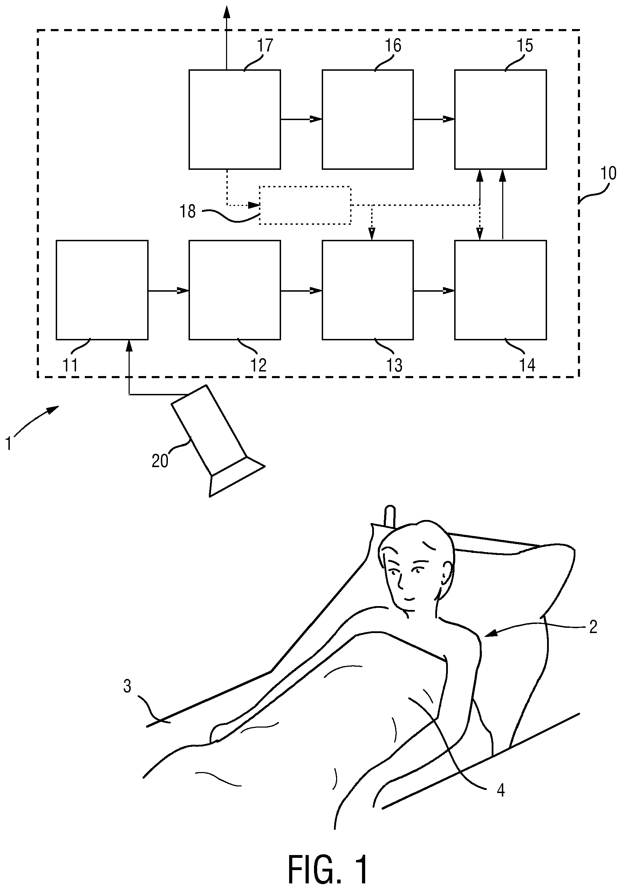

Fig. 1 shows a schematic diagram of system 1 and a device 10 for determining vital sign information of a subject 100. The subject 2 in this example is a patient lying in a bed 3, e.g. in a hospital or other healthcare facility, but may also be a neonate or premature infant, e.g. lying in an incubator, or person at home or in a different environment. Besides the device 10 the system 1 generally comprises an imaging unit 20, such as a camera, for acquiring image data of the subject 2, said image data including a sequence of images over time.

The image frames captured by the imaging unit 20 may particularly correspond to a video sequence captured by means of an analog or digital photosensor, e.g. in a (digital) camera. Such a camera usually includes a photosensor, such as a CMOS or CCD sensor, which may also operate in a specific spectral range (visible, IR) or provide information for different spectral ranges. The camera may provide an analog or digital signal.

The device 10 comprises an input unit 11 for receiving the image data of the subject 2, either directly from the imaging unit 20 or from a storage, in which image data acquired earlier have been stored or buffered.

In an ROI selection unit 12 an initial region of interest (ROI) is selected within an image included in said image data. This can be done in different ways, e.g. manually (via an interface) by the user or automatically, e.g. based on one of the known methods mentioned above, such as the method disclosed in WO 2014/131850 A1.

A feature selection unit 13 selects one or more spatial features of a body part within the initial ROI. A spatial feature is generally a combination of neighboring pixels, which is distinctive from other combinations of neighboring pixels in the vicinity, and which stays distinctive even after scaling or motion of a pixel area. The spatial features are preferably easily identifiable features or feature points shown within the initial ROI, which can be tracked through the images of the image data. Examples of spatial features are corners, edges or lines (e.g. of a pattern printed onto the subject's clothes or the blanket) or anatomic features such as eyes or nose.

A motion signal extraction unit 14 is provided for extracting from said image data within the initial ROI the direction and/or amplitude (or amplitude range) of motion of a subject's body part related to a desired vital sign of the subject. For instance, if breathing motion of the chest area 4 of the subject 2 shall be monitored, the direction and/or amplitude of this breathing motion is determined, e.g. by use of pattern recognition or other image processing means. In case of breathing motion mostly vertical and horizontal breathing motion components may be determined. In other embodiments the heartbeat-related motion of the chest, carotid or head may be determined. In case a person is sitting in front of a camera, heartbeat would mostly be associated with motion of a head along vertical axis. That means that any (combination of) spatial feature which is invariant to vertical motion can be selected. For instance, a distance between eyes will not change during vertical motion of a head, but will change during head rotation.

Further, a detection unit 15 is provided for detecting one or more spatial features of said selected spatial features, whose motion is not related to the desired vital sign of the subject. Hence, it is distinguished between motion of spatial features which is related to the desired vital sign (e.g. movements of the chest caused by breathing or heartbeat, such as lifting and lowering of the chest wall and the abdominal wall due to breathing) and which is not related to the desired vital sign (e.g. movements of the chest e.g. in lateral or rotational direction due to motion of the patient, such as rolling or shifting of the whole body). Preferred embodiments for implementing the function of the detection unit will be explained below.

A tracking unit 16 is provided for tracking the initial ROI based on changes of the position of said one or more detected spatial features within the initial ROI, to obtain a final ROI. In other words, the spatial features with are unrelated with the desired vital sign and whose position is not influenced by the motion related with the desired vital sign (such as heartbeat motion or breathing motion) are used to determine if - over time - a motion of the body part (or even the whole body) of the subject appears which is not caused by a motion related with the desired vital sign. This information is then used to adapt the position of the initial ROI accordingly, i.e. to shift the initial ROI according to the detected motion (which is not related to the desired vital sign) to a new position, said shifted ROI representing the final ROI.

Finally, in a vital signs extraction unit 17 the desired vital sign, e.g. respiratory information, such as respiration rate, and/or heartbeat information, such as pulse rate, is extracted from the final ROI. The vital sign is particularly derived from the motion of the body part within the final ROI (or within the ROI as continuously tracked in the above described way), e.g. by determining the frequency of lifting and lowering of the chest wall and/or the abdominal wall caused by breathing or by determining the frequency of tiny head movements (up and down) caused by heartbeat.

The extracted vital sign can then either be further processed or issued, e.g. on a display of a patient monitor, a central monitoring station (e.g.in a nurse room), a handheld monitoring device (e.g. a smartphone of a nurse or doctor), etc.

Preferably, the device 10 may further comprise an ROI re-initialization unit 18, as indicated with broken lines in Fig. 1, for re-initializing the final ROI as new initial ROI. Hence, the monitoring and tracking of the ROI as explained above may be iteratively carried out to further improve the accuracy and reliability of the extracted vital sign(s). Hence, after re-initialization of the initial ROI the processing preferably continues with the selection of one or more spatial features of a body part within the new initial ROI as performed by the feature selection unit 13. In an embodiment the same spatial features as used in one or more previous iterations are used again so that the processing may also continue with the step carried out by the motion signal extraction unit 14 or the detection unit 15.

It shall be noted that one or more of the elements of the device 1 can be implemented by dedicated hardware, software or a combination thereof. For instance, in an embodiment the elements are implemented by processing means, such as a processor or computer, which are programmed accordingly.

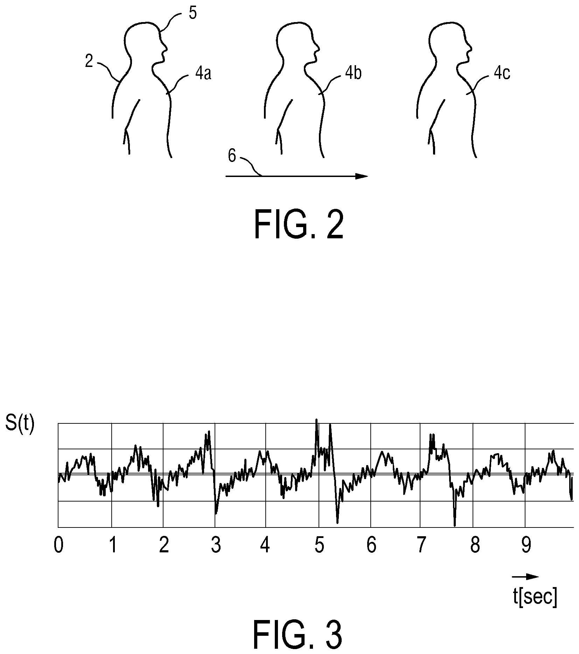

Fig. 2 shows a schematic illustration of the subject for illustrating the remote measurement of respiratory information, such as the respiration rate, of the subject 2. The subject 2 undergoes a characteristic motion of an indicative portion 4 (such as the chest) due to the respiration. When breathing, expansion and contraction of the lungs causes slight motion of characteristic portions of living beings, e.g. lifting and lowering of the chest 4. Also, abdominal breathing can cause characteristic motion of respective parts of the subject's body. At least partially periodic motion patterns included by physiological processes can occur in many living beings, particularly in human beings or animals.

Over time, as indicated by an arrow 6, the indicative portion 4 is moved between a contracted position, indicated by reference numerals 4a, 4c, and an extracted portion, indicated by 4b. Essentially, based on this motion pattern, for instance the respiration rate or respiration rate variability can be assessed by means of pattern or edge detection in a captured image sequence. While the indicative portion 4 is pulsating over time, other non-indicative portions such as the head 5 remain substantially motionless (unless otherwise moved by the subject 2), i.e. the non-indicative portions also undergo diverse motion over time. However, these motions do not correspond to the periodic pulsation of the chest 4, i.e. are not related to the desired vital sign (respiration information in this example) and shall be distinguished there from.

Fig. 3 shows a time diagram of an alternating signal S over time derived from the movement pattern and/or from motion vectors of the ROI which can be for example determined on the basis of a frame or an edge detection. The alternating signal S in this particular example corresponds to the movement of the chest 4 of the subject 2 derived from a ROI with the sequence of images of the image data. The alternating signal S shows a characteristic variation corresponding to the movement of the chest 4 i.e. the breathing rate of the subject 2. The alternating signal S also shows a high-frequency noise superimposed to the breathing rate. By use of a frequency analysis the frequency of the breathing motion, i.e. the breathing rate, can be determined.

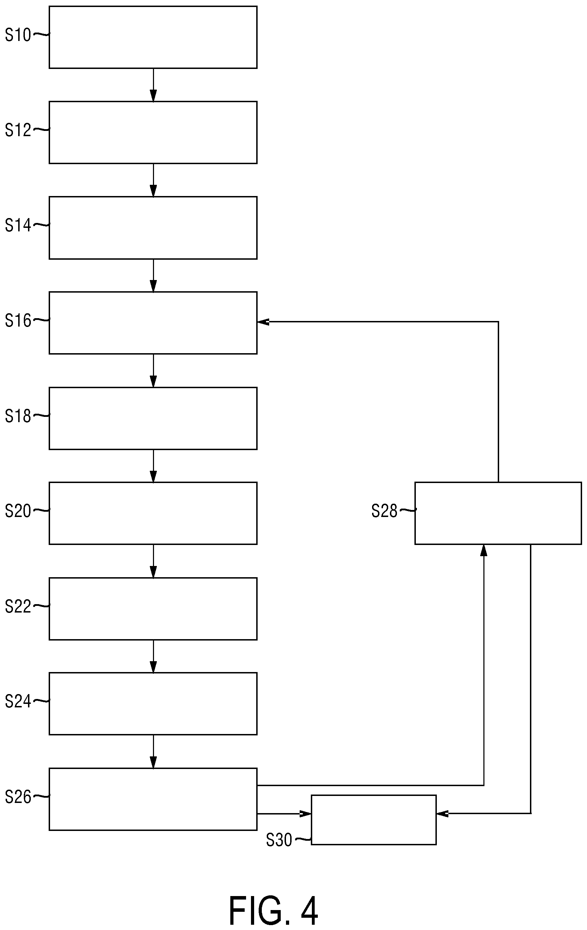

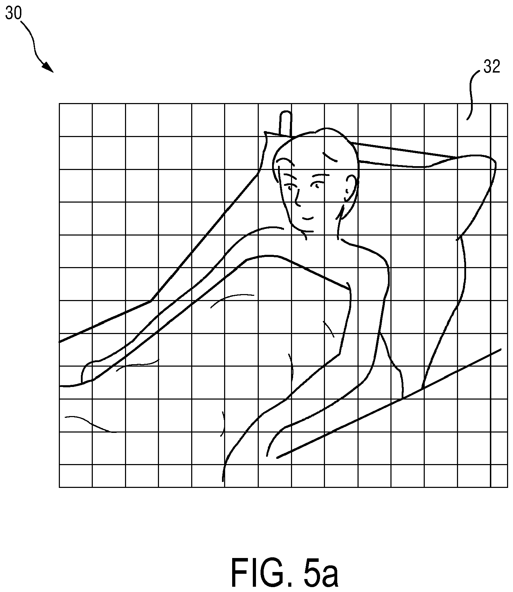

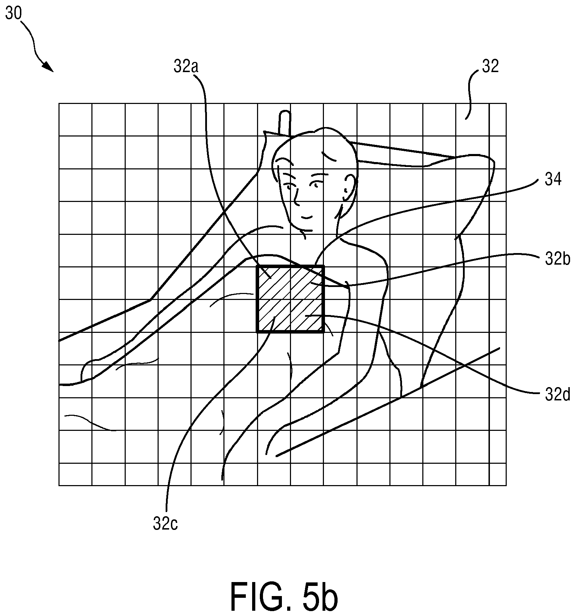

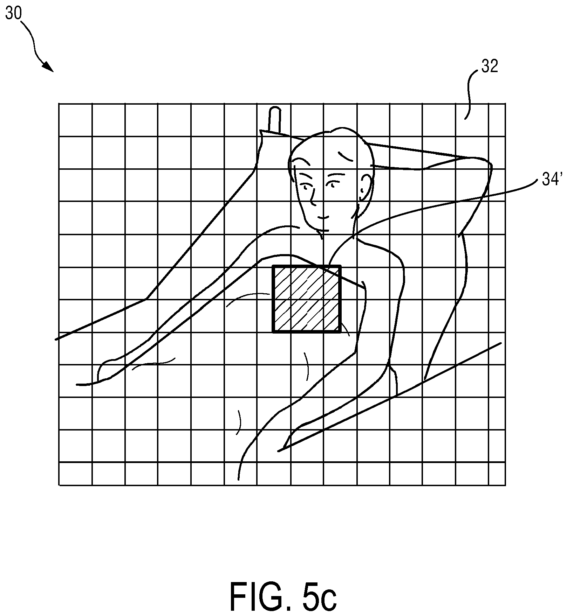

Fig. 4 shows a flow chart of a preferred embodiment of the method 100 according to the present invention. Figs. 5a, 5b, and 5c show a schematic image sequence for illustrating the steps of this embodiment of the method 100.

In a first step S10 an image 30 is divided into a plurality of spatial blocks 32 as shown in Fig. 5a. In a second step S12 a vital sign signal (e.g. respiratory motion) is extracted from a plurality or even each of the blocks 32. In a third step S14 adjacent spatial blocks with the strongest extracted signal (according to predefined criteria such as amplitude, SNR, frequency, or a shape of a signal) are clustered as shown in Fig. 5b, in which the clustered spatial blocks 32a-32d are indicated representing an initial ROI 34.

In a fourth step S16 strong spatial features are selected inside the initial ROI 34, which will be used for tracking. Such strong spatial features may be edges of the blanket in the direction of breathing motion, dynamic range of pixels, orientation of the edge of a blanket, any other features which are not changed by respiratory motion. In case of the edge of a blanket: the edge can move with each respiratory motion, while its orientation would not be changed by a respiratory motion, but by any other body motion (e.g. rotation of a body).

In a fifth step S18 one or more vital signs are extracted from the entire initial ROI 34, e.g. by detecting motion of the chest caused by breathing of the subject 2 as explained above. Based on the extracted vital sign(s) the main direction (e.g. mostly vertical or horizontal breathing motion component) and the amplitude range of the extracted vital sign signal are determined in a sixth step S20. That can be done by analyzing the amplitude of motion induced by a vital sign (respiration or pulse) along all possible directions, and select the one direction, which correlates the most with the vital signs motion.

In a seventh step S22 it is controlled whether the initial ROI 34 is moving, e.g. by analyzing the changes of distances between spatial features (or feature points) inside the initial ROI 34, the direction of changes of spatial features and/or the deviation of motion of spatial features from the main characteristics of the extracted vital sign signal.

In case a motion of the initial ROI 34 as detected in step S22, a motion estimation algorithm is started and the spatial features detected inside the initial ROI 34 are tracked in an eighth step S24. If the new position of the spatial features becomes stable over several frames, extraction of the vital signs signal, e.g. the respiratory signal, from this new (final) ROI is started in a ninth step S26. The position of the new (final) ROI 34' is shown in Fig. 5c, illustrating that the patient has moved a bit to the right side with respect to the bed so that also the initial ROI is shifted by the same amount and to the same side.

Finally, in a tenth step S28 the spatial blocks around the new location of the ROI are analyzed, e.g. using the same approach as used in the third step S14, and the new (final) ROI is re-initialized as new initial ROI. The procedure may then be carried out iteratively, with step S16 being the next step. Further, in a tenth step S30 the desired vital sign information can be derived from the new (final) ROI.

A vital sign signal may be derived in the second steps S12 as follows. First, movement patterns are derived from a plurality or each of the image blocks 32 of the image frame 30 and the alternating signals S are determined from motion vectors determined from the movement pattern of each of the image blocks 32 as described above. The motion vectors may be determined by pattern detection or edge detection within the different image blocks 32. On the basis of the frequency analysis performed e.g. by a frequency analysis unit, it is determined whether the movement pattern of the different image blocks 32 corresponds to vital sign information or whether the movement patterns are disturbance signals or noise. The determination whether the movement patterns include vital sign information or not may be performed on the basis of the spectral parameter and/or the spectral energy and whether the spectral energy in a predefined frequency band is larger than a predefined percentage of the entire spectral energy of the respective alternating signal.

In the fourth step S16 spatial image features may be selected, which are used for detection of non-vital-sign-related body motion and reliable tracking of the ROI. Therefore, those image features should be the most (temporally) stable during vital-sign-related motion, such as respiratory motion in case of extracting respiratory information as vital sign information. For instance, if a monitored subject is sitting in front of a camera, and a respiratory motion has a strong vertical motion component, the image features selected inside the ROI should be least affected (ideally invariant) to vertical motion, e.g. vertical edges.

Generally, the vital sign signal extracted at step S26 might have a lower quality than a vital sign signal extracted at step S30. At the same time, this approach allows restart of extraction of vital signs immediately after the motion of a subject is completed, without the need to trigger a complete ROI detection algorithm (as performed in this embodiment in steps S12 and S14), which would reduce the gaps in monitoring of vital signs during motion and eventually improve the responsiveness of the entire device and method. An additional advantage of this approach is that the vital sign signal is extracted only from the stable ROI, identified during steps S12 and S14. Thus, any disturbances, or other subjects with the similar respiratory motion would not influence measurements.

In another embodiment of the present invention, step S28 (re-selection of spatial areas around the previous ROI) can be initialized every time when the reliability or quality of the extracted signals drops below a certain threshold.

In yet another embodiment of the present invention, the steps S20 and S22 can include a method for detection of (partial) occlusion of ROI, which would trigger the step S26 for extraction of the vital sign signal from non-occluded parts within the initial ROI. Among other methods, the occlusion detection can be done by analyzing the visibility of spatial features, identified in the previous steps.

In summary, the present invention applies two different modalities (i.e. vital signs based and spatial features based) in a particular combination for an advantageous selection and tracking of the ROI. The detection of non-respiratory (in general non-vital-sign-related) body motion is based on an analysis of image features inside the ROI, rather than on an analysis of the extracted vital sign signal. Further, tracking of the ROI used for vital sign monitoring is based on motion analysis of image features inside the ROI, which are least affected by the vital-sign-related motion, such as the respiratory motion.

The present invention thus uses (re-)initialization and tracking of ROI, based on the combination of temporal features and spatial features. The spatial features (e.g. the direction of edges, pixel patterns) are detected only within an area, initialized by strong vital-sign-related motion (e.g. strong breathing motion), and only those features (e.g. angle of an edge), which are not deformed by the vital-sign-related motion are used for maintaining or tracking of ROI, even in the absence of the vital sign signal, e.g. in the absence of respiration or a pulse signal.

Further, the decision to re-initialize ROI is based on analysis of displacement of spatial features, rather than based only on changes in a breathing temporal pattern. Therefore, the ROI will not be re-initialized if a subject stops breathing. At the same time, motion of a subject will be detected and ROI will be tracked (based on tracking of spatial features) even if a temporal breathing pattern does not change, but the spatial features are deformed. The spatial features should be invariant to the vital-sign-related motion (e.g. direction of an edge in ROI, which does not change due to respiratory motion). Hence, any change in those spatial features would indicate non-vital-sign-related motion, e.g. body motion, of the ROI. An example of such features is that a vertical edge of pixels in the presence of vertical breathing will not be affected by breathing, but will be displaced by even slight rotation of a body in a horizontal direction.

The present invention may primarily be applied in respiration monitoring devices and method within hospitals or in home care and sports.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device for detecting vital sign information of a subject (2), comprising:
- an input unit (11) for receiving image data of the subject, said image data including a sequence of images over time,
- an ROI selection unit (12) for selecting an initial region of interest, ROI, within an image of the sequence of images, said initial ROI comprising a body part of the subject,
- a feature selection unit (13) for selecting one or more spatial features of the body part of the subject within the initial ROI,
- a motion signal extraction unit (14) for extracting from said image data within the initial ROI the direction and/or amplitude of motion of the body part related to a desired vital sign of the subject,
- a detection unit (15) for detecting one or more spatial features of said selected spatial features, whose motion is not related to the desired vital sign of the subject, based on the extracted direction and/or amplitude of motion of the body part,
- a tracking unit (16) for tracking the initial ROI within other images of the sequence of images, by detecting changes of the position of said one or more detected spatial features and by shifting the position of the initial ROI based on the detected changes to obtain corresponding final ROIs within said other images, and
- a vital signs extraction unit (17) for extracting the desired vital sign from the final ROIs within images of said sequence of images.

2. Device as claimed in claim 1, further comprising an ROI re-initialization unit (18) for re-initializing a final ROI as new initial ROI.

3. Device as claimed in claim 1, wherein said tracking unit (16) is configured to use as final ROI the ROI obtained from tracking of the initial ROI after the position of the one or more spatial feature has become stable over several image frames.

4. Device as claimed in claim 1,
wherein said feature selection unit (13) is configured to select one or more spatial features within the initial ROI, which are invariant, preferably most invariant, to motion of the body part related to a desired vital sign of the subject.

5. Device as claimed in claim 1,
wherein the motion of the body part related to a desired vital sign of the subject is breathing motion and wherein the vital sign information is respiratory information.

6. Device as claimed in claim 1,
wherein the motion of the body part related to a desired vital sign of the subject is heartbeat motion and wherein the vital sign information is pulse rate information.

7. Device as claimed in claim 5 or 6,
wherein said feature selection unit (13) is configured to select one or more spatial features within the initial ROI, which are invariant, in particular most invariant, to breathing motion or heartbeat motion, in particular spatial features showing edges or lines in an image within the initial ROI arranged along the main direction of said breathing motion or heartbeat motion within the initial ROI.

8. Device as claimed in claim 1,
wherein said detection unit (15) is configured to detect one or more spatial features of said selected spatial features, whose motion is not related to the desired vital sign of the subject, by detecting changes of the position of said selected spatial features within the initial ROI and the extracted direction and/or amplitude of motion of the body part related to a desired vital sign of the subject.

9. Device as claimed in claim 1,
wherein said detection unit (15) is configured to detect one or more spatial features of said selected spatial features, whose motion is not related to the desired vital sign of the subject, by analyzing changes of distances between selected spatial features within the initial ROI, the direction of changes of the position of selected spatial features within the initial ROI and/or the deviation of changes of the position of selected spatial features within the initial ROI from the motion of the body part related to a desired vital sign of the subject.

10. Device as claimed in claim 1,
wherein said ROI selection unit (12) is configured to select the initial ROI within an image by dividing the image into spatial blocks, extracting a vital sign from a plurality of said spatial blocks, clustering of two or more adjacent spatial blocks, from which the strongest vital signs are extracted, and selecting said clustered spatial blocks as initial ROI.

11. Device as claimed in claim 1,
wherein said ROI selection unit (12) is configured to select the initial ROI from a previously obtained final ROI by extracting a vital sign from a plurality of spatial blocks adjacent to the obtained final ROI, clustering of two or more adjacent spatial blocks, from which the strongest vital signs are extracted, and selecting said clustered spatial blocks as new initial ROI.

12. Device as claimed in claim 11,
wherein said ROI selection unit (12) is configured to select the initial ROI from a previously obtained final ROI if the reliability or quality of the vital sign from the final ROI drops below a predetermined threshold or by predetermined amount.

13. Device as claimed in claim 1,
wherein said motion detection unit (15) is configured to detect if the initial ROI is at least partly occluded and wherein said vital signs extraction unit (17) is configured to extracting the desired vital sign from non-occluded parts of the initial ROI.

14. Method for detecting vital sign information of a subject (2), comprising:
- receiving image data of the subject, said image data including a sequence of images over time,
- selecting an initial region of interest, ROI, within an image, said initial ROI comprising a body part of the subject,
- selecting one or more spatial features of the body part of the subject within the initial ROI,
- extracting from said image data within the initial ROI the direction and/or amplitude of motion of the body part related to a desired vital sign of the subject,
- detecting one or more spatial features of said selected spatial features, whose motion is not related to the desired vital sign of the subject, based on the extracted direction and/or amplitude of motion of the body part,
- tracking the initial ROI within other images of the sequence of images by detecting changes of the position of said one or more detected spatial features and by shifting the position of the initial ROI based on the detected changes to obtain corresponding final ROIs within said other images, and
- extracting the desired vital sign from the final ROIs within images of said sequence of images.

15. Computer program comprising program code means for causing a computer to carry out the steps of the method as claimed in claim 14 when said computer program is carried out on the computer.

## Patentansprüche

1. Vorrichtung zum Detektieren von Lebenszeicheninformationen eines Patienten (2), umfassend:
- eine Eingangseinheit (11) zum Empfangen von Bilddaten des Patienten, wobei die genannten Bilddaten eine Bildfolge über die Zeit umfasst,
- eine ROI-Auswahleinheit (12) zum Auswählen einer anfänglichen interessierenden Region (region of interest, ROI) in einem Bild der Bildfolge, wobei die anfängliche ROI einen Körperteil des Patienten umfasst,
- eine Merkmalauswahleinheit (13) zum Auswählen von einem oder mehreren räumlichen Merkmalen des Körperteils des Patienten in der anfänglichen ROI,
- eine Bewegungssignal-Extraktionseinheit (14), um die Richtung und/oder Amplitude der in Zusammenhang mit einem gewünschten Lebenszeichen des Patienten stehenden Bewegung des Körperteils aus den genannten Bilddaten in der anfänglichen ROI zu extrahieren,
- eine Detektionseinheit (15), um basierend auf der extrahierten Richtung und/oder Amplitude der Bewegung des Körperteils ein oder mehrere räumliche Merkmale der genannten ausgewählten räumlichen Merkmale zu detektieren, deren Bewegung nicht mit dem gewünschten Lebenszeichen des Patienten in Zusammengang steht,
- eine Verfolgungseinheit (16), um die anfängliche ROI in anderen Bildern der Bildfolge zu verfolgen, indem sie Positionsänderungen der genannten einen oder mehreren detektierten räumlichen Merkmale detektiert und die Position der anfänglichen ROI basierend auf den detektierten Änderungen verschiebt, um entsprechende endgültige ROIs in den genannten anderen Bildern zu erlangen, und
- eine Lebenszeichen-Extraktionseinheit (17), um das gewünschten Lebenszeichen aus den endgültigen ROIs in Bildern der genannten Bildfolge zu extrahieren.

2. Vorrichtung nach Anspruch 1,
weiterhin umfassend eine ROI-Reinitialisierungseinheit (18) zu Reinitialisieren einer endgültigen ROI als neue anfängliche ROI.

3. Vorrichtung nach Anspruch 1,
wobei die genannte Verfolgungseinheit (16) konfiguriert ist, um die aus dem Verfolgen der anfänglichen ROI erlangte ROI als endgültige ROI zu verwenden, nachdem die Position des einen oder mehrerer räumlicher Merkmale über mehrere Einzelbilder stabil geworden ist.

4. Vorrichtung nach Anspruch 1,
wobei die genannte Merkmalauswahleinheit (13) konfiguriert ist, um ein oder mehrere räumliche Merkmale in der anfänglichen ROI auszuwählen, die invariant, vorzugsweise äußerst invariant, gegenüber der im Zusammenhang mit einem gewünschten Lebenszeichen des Patienten stehenden Bewegung des Körperteils sind.

5. Vorrichtung nach Anspruch 1,
wobei die in Zusammengang mit einem gewünschten Lebenszeichen des Patienten stehende Bewegung des Körperteils Atembewegung ist und wobei die Lebenszeicheninformationen Atmungsinformationen sind.

6. Vorrichtung nach Anspruch 1,
wobei die in Zusammenhang mit einem gewünschten Lebenszeichen des Patienten stehende Bewegung Herzschlagbewegung ist und wobei die Lebenszeicheninformationen Pulsfrequenzinformationen sind.

7. Vorrichtung nach Anspruch 5 oder 6,
wobei die genannte Merkmalauswahleinheit (13) konfiguriert ist, um ein oder mehrere räumliche Merkmale in der anfänglichen ROI auszuwählen, die invariant, insbesondere äußerst invariant, gegenüber Atembewegung oder Herzschlagbewegung sind, insbesondere räumliche Merkmale mit Rändern oder Linien in einem Bild in der anfänglichen ROI, die entlang der Hauptrichtung der genannten Atembewegung oder Herzschlagbewegung in der anfänglichen ROI angeordnet sind.

8. Vorrichtung nach Anspruch 1,
wobei die genannte Detektionseinheit (15) konfiguriert ist, um ein oder mehrere räumliche Merkmale der genannten ausgewählten räumlichen Merkmale zu detektieren, deren Bewegung nicht in Zusammenhang mit dem gewünschten Lebenszeichen des Patienten steht, indem Positionsänderungen der genannten ausgewählten räumlichen Merkmale in der anfänglichen ROI und die extrahierte Richtung/oder Amplitude der in Zusammenhang mit einem gewünschten Lebenszeichen des Patienten stehenden Bewegung der Körperteils detektiert werden.

9. Vorrichtung nach Anspruch 1,
wobei die genannte Detektionseinheit (15) konfiguriert ist, um ein oder mehrere räumliche Merkmale der genannten ausgewählten räumlichen Merkmale zu detektieren, deren Bewegung nicht in Zusammenhang mit dem gewünschten Lebenszeichen des Patienten steht, indem Änderungen der Abstände zwischen ausgewählten räumlichen Merkmalen in der anfänglichen ROI, die Richtung von Positionsänderungen von ausgewählten räumlichen Merkmalen in der ausgewählten ROI und/oder die Abweichung von Positionsänderungen von ausgewählten räumlichen Merkmalen in der anfänglichen ROI aus der in Zusammenhang mit einem gewünschten Lebenszeichen des Patienten stehenden Bewegung des Körperteils analysiert werden.

10. Vorrichtung nach Anspruch 1,
wobei die genannte ROI-Auswahleinheit (12) konfiguriert ist, um die anfängliche ROI in einem Bild auszuwählen, indem das Bild in räumliche Blöcke unterteilt wird, ein Lebenszeichen aus einer Vielzahl von genannten räumlichen Blöcken extrahiert wird, zwei oder mehr benachbarte räumliche Blöcke zusammengefasst werden, aus denen die stärksten Lebenszeichen extrahiert werden, und die genannten zusammengefassten räumlichen Blöcke als anfängliche ROI ausgewählt werden.

11. Vorrichtung nach Anspruch 1,
wobei die genannte ROI-Auswahleinheit (12) konfiguriert ist, um die anfängliche ROI aus einer zuvor erlangten endgültigen ROI auszuwählen, indem ein Lebenszeichen aus einer Vielzahl von an die erlangte endgültige ROI angrenzenden räumlichen Blöcken extrahiert wird, zwei oder mehr benachbarte räumliche Blöke zusammengefasst werden, aus denen die stärksten Lebenszeichen extrahiert werden, und die genannten zusammengefassten räumlichen Blöcke als neue anfängliche ROI ausgewählt werden.

12. Vorrichtung nach Anspruch 11,
wobei die genannte ROI-Auswahleinheit (12) konfiguriert ist, um die anfängliche ROI aus einer zuvor erlangten endgültigen ROI auszuwählen, wenn die Zuverlässigkeit oder Qualität des Lebenszeichens aus der endgültigen ROI unter einen vorgegebenen Schwellenwert oder um einen vorgegebenen Betrag fällt.

13. Vorrichtung nach Anspruch 1,
wobei die genannte Bewegungsdetektionseinheit (15) konfiguriert ist, um zu detektieren, ob die anfängliche ROI mindestens teilweise verdeckt ist und wobei die genannte Lebenszeichen-Extraktionseinheit (17) konfiguriert ist, um das gewünschte Lebenszeichen aus nicht-verdeckten Teilen der anfänglichen ROI zu extrahieren.

14. Verfahren zum Detektieren von Lebenszeicheninformationen eines Patienten (2), umfassend:
- Empfangen von Bilddaten des Patienten, wobei die genannten Bilddaten eine Bildfolge über die Zeit umfassen,
- Auswählen einer anfänglichen interessierenden Region (region of interest, ROI) in einem Bild, wobei die anfängliche ROI einen Körperteil des Patienten umfasst,
- Auswählen von einem oder mehreren räumlichen Merkmalen des Körperteils des Patienten in der anfänglichen ROI,
- Extrahieren der Richtung und/oder Amplitude der in Zusammenhang mit einem gewünschten Lebenszeichen des Patienten stehenden Bewegung des Körperteils aus den genannten Bilddaten in der anfänglichen ROI,
- Detektieren von einem oder mehreren räumlichen Merkmalen der genannten ausgewählten räumlichen Merkmale, deren Bewegung nicht mit dem gewünschten Lebenszeichen des Patienten in Zusammengang steht, basierend auf der extrahierten Richtung und/oder Amplitude der Bewegung des Körperteils,
- Verfolgen der anfänglichen ROI in anderen Bildern der Bildfolge durch Detektieren von Positionsänderungen der genannten einen oder mehreren detektierten räumlichen Merkmale und durch Verschieben der Position der anfänglichen ROI basierend auf den detektierten Änderungen, um entsprechende endgültige ROIs in den genannten anderen Bildern zu erlangen, und
- Extrahieren des gewünschten Lebenszeichens aus den endgültigen ROIs in Bildern der genannten Bildfolge.

15. Computerprogramm umfassend Programmcodemittel zum Veranlassen eines Computers, die Schritte des Verfahrens wie in Anspruch 14 beansprucht durchzuführen, wenn das genannte Computerprogramm auf dem Computer ausgeführt wird.

## Revendications

1. Dispositif pour détecter des informations relatives aux signes vitaux d'un sujet (2), comprenant :
- une unité d'entrée (11) destinée à recevoir des données d'images du sujet, lesdites données d'images comprenant une séquence d'images dans le temps,
- une unité (12) de sélection des régions d'intérêt ROI destinée à sélectionner une région d'intérêt initiale, ROI, à l'intérieur d'une image de la séquence d'images, ladite ROI initiale comprenant une partie du corps du sujet,
- une unité de sélection de caractéristiques (13) destinée à sélectionner une ou plusieurs caractéristiques spatiales de la partie du corps du sujet à l'intérieur de la ROI initiale,
- une unité (14) d'extraction de signaux de mouvement destinée à extraire desdites données d'image à l'intérieur de la ROI initiale la direction et/ou l'amplitude du mouvement de la partie du corps associé à un signe vital souhaité du sujet,
- une unité de détection (15) destinée à détecter une ou plusieurs caractéristiques spatiales desdites caractéristiques spatiales sélectionnées, dont le mouvement n'est pas associé au signe vital souhaité du sujet, sur la base de la direction et/ou de l'amplitude extraites du mouvement de la partie du corps,
- une unité de suivi (16) destinée à suivre la ROI initiale à l'intérieur d'autres images de la séquence d'images, en détectant des changements de la position desdites une ou plusieurs caractéristiques spatiales détectées et en modifiant la position de la ROI initiale en fonction des changements détectés pour obtenir des ROI finales correspondantes à l'intérieur desdites autres images, et
- une unité (17) d'extraction de signes vitaux destinée à extraire le signe vital souhaité des ROI finales à l'intérieur d'images de ladite séquence d'images.

2. Dispositif selon la revendication 1,
comprenant en outre une unité (18) de réinitialisation de ROI destinée à réinitialiser une ROI finale en tant que nouvelle ROI finale.

3. Dispositif selon la revendication 1,
dans lequel ladite unité de suivi (16) est configurée pour utiliser comme ROI finale la ROI obtenue à partir du suivi de la ROI initiale une fois que la position des une ou plusieurs caractéristiques spatiales s'est stabilisée sur plusieurs trames d'image.

4. Dispositif selon la revendication 1,
dans lequel ladite unité de sélection de caractéristiques (13) est configurée pour sélectionner une ou plusieurs caractéristiques spatiales à l'intérieur de la ROI initiale, qui sont insensibles, de préférence complètement insensibles, à un mouvement de la partie du corps associé à un signe vital souhaité du sujet.

5. Dispositif selon la revendication 1,
dans lequel le mouvement de la partie du corps associé à un signe vital souhaité du sujet est un mouvement de respiration et dans lequel les informations relatives à un signe vital sont des informations respiratoires.

6. Dispositif selon la revendication 1,
dans lequel le mouvement de la partie du corps associé à un signe vital souhaité du sujet est un mouvement de battement cardiaque et dans lequel les informations relatives à un signe vital sont des informations de fréquence de pouls.

7. Dispositif selon la revendication 5 ou 6,
dans lequel ladite unité de sélection de caractéristiques (13) est configurée pour sélectionner une ou plusieurs caractéristiques spatiales à l'intérieur de la ROI initiale, qui sont qui sont insensibles, de préférence complètement insensibles, à un mouvement de respiration ou à un mouvement de battement cardiaque, en particulier des caractéristiques spatiales présentant des bords ou des lignes dans une image à l'intérieur de la ROI initiale agencés le long de la direction principale dudit mouvement de respiration ou mouvement de battement cardiaque à l'intérieur de la ROI initiale.

8. Dispositif selon la revendication 1,
dans lequel ladite unité de détection (15) est configurée pour détecter une ou plusieurs caractéristiques spatiales desdites caractéristiques spatiales sélectionnées, dont le mouvement n'est pas associé au signe vital souhaité du sujet, en détectant des changements de la position desdites caractéristiques spatiales sélectionnées à l'intérieur de la ROI initiale et de la direction et/ou de l'amplitude extraites du mouvement de la partie du corps associé à un signe vital souhaité du sujet.

9. Dispositif selon la revendication 1,
dans lequel ladite unité de détection (15) est configurée pour détecter une ou plusieurs caractéristiques spatiales desdites caractéristiques spatiales sélectionnées, dont le mouvement n'est pas associé au signe vital souhaité du sujet, en analysant des changements de distances entre des caractéristiques spatiales sélectionnées à l'intérieur de la ROI initiale, la direction des changements de la position des caractéristiques spatiales sélectionnées à l'intérieur de la ROI initiale et/ou l'écart des changements de la position de caractéristiques spatiales sélectionnées à l'intérieur de la ROI initiale à partir du mouvement de la partie du corps associé à un signe vital souhaité du sujet.

10. Dispositif selon la revendication 1,
dans lequel ladite unité de sélection des ROI (12) est configurée pour sélectionner la ROI initiale à l'intérieur d'une image en divisant l'image en blocs spatiaux, en extrayant un signe vital d'une pluralité desdits blocs spatiaux, en regroupant deux blocs spatiaux adjacents ou plusieurs, à partir desquels les signes vitaux les plus forts sont extraits, et en sélectionnant lesdits blocs spatiaux regroupés en tant que ROI initiale.

11. Dispositif selon la revendication 1,
dans lequel ladite unité de sélection des ROI (12) est configurée pour sélectionner la ROI initiale à partir d'une ROI finale précédemment obtenue en extrayant un signe vital d'une pluralité de blocs spatiaux adjacents à la ROI final obtenue, par regroupement de deux blocs spatiaux adjacents ou plusieurs, à partir desquels les signes vitaux les plus forts sont extraits, et en sélectionnant lesdits blocs spatiaux regroupés en tant que nouvelle ROI initiale.

12. Dispositif selon la revendication 11,
dans lequel ladite unité de sélection des ROI (12) est configurée pour sélectionner la ROI initiale à partir d'une ROI finale précédemment obtenue si la fiabilité ou la qualité du signe vital provenant de la ROI finale baisse en dessous d'un seuil prédéterminé ou d'une quantité prédéterminée.

13. Dispositif selon la revendication 1,
dans lequel ladite unité de détection de mouvement (15) est configurée pour détecter si la ROI initiale est au moins partiellement obstruée et dans lequel ladite unité (17) d'extraction de signes vitaux est configurée pour extraire le signe vital souhaité des parties non obstruées de la ROI initiale.

14. Procédé pour détecter des informations relatives aux signes vitaux d'un sujet (2), comprenant :
- la réception de données d'images du sujet, lesdites données d'images comprenant une séquence d'images dans le temps,
- la sélection d'une région d'intérêt initiale, ROI, à l'intérieur d'une image, ladite ROI initiale comprenant une partie du corps du sujet,
- la sélection d'une ou plusieurs caractéristiques spatiales de la partie du corps du sujet à l'intérieur de la ROI initiale,
- l'extraction desdites données d'image à l'intérieur de la ROI initiale de la direction et/ou de l'amplitude du mouvement de la partie du corps associé à un signe vital souhaité du sujet,
- la détection d'une ou plusieurs caractéristiques spatiales desdites caractéristiques spatiales sélectionnées, dont le mouvement n'est pas associé au signe vital souhaité du sujet, sur la base de la direction et/ou de l'amplitude extraites du mouvement de la partie du corps,
- le suivi de la ROI initiale à l'intérieur d'autres images de la séquence d'images par détection de changements de la position desdites une ou plusieurs caractéristiques spatiales détectées et par modification de la position de la ROI initiale en fonction des changements détectés pour obtenir des ROI finales correspondantes à l'intérieur desdites autres images, et
- l'extraction du signe vital souhaité des ROI finales à l'intérieur d'images de ladite séquence d'images.

15. Programme informatique comprenant des moyens de code de programme destinés à amener un ordinateur à effectuer les étapes du procédé selon la revendication 14 lorsque ledit programme informatique est exécuté sur l'ordinateur.
